# EUROPEAN PATENT SPECIFICATION

(11) **EP 2 601 905 B1**
(45) Date of publication and mention of the grant of the patent: **18.02.2015**
(21) Application number: 11823285.9
(22) Date of filing: 25.03.2011
(51) Int. Cl.: A61B 19/00, B25J 9/16

(54) **CONTROL APPARATUS OF MANIPULATOR SYSTEM, MANIPULATOR SYSTEM, AND CONTROL METHOD OF MANIPULATOR SYSTEM**
STEUERVORRICHTUNG FÜR EIN MANIPULATORSYSTEM, MANIPULATORSYSTEM UND STEUERVERFAHREN FÜR DAS MANIPULATORSYSTEM
DISPOSITIF DE COMMANDE DE SYSTÈME MANIPULATEUR, SYSTÈME MANIPULATEUR ET PROCÉDÉ DE COMMANDE DE SYSTÈME MANIPULATEUR

(30) Priority: 10.09.2010 JP 2010203506
(43) Date of publication of application: 12.06.2013
(73) Proprietor: OLYMPUS CORPORATION, Shibuya-ku Tokyo 151-0072 (JP)
(72) Inventor: NAMIKI, Hirotaka, Hachioji-shi Tokyo 192-8512 (JP)
(74) Representative: Gunzelmann, Rainer
(86) International application number: PCT/JP2011/057338
(87) International publication number: WO 2012/032806

(56) References cited:
- JP-A- 2004 105 451
- JP-A- 2009 090 383
- JP-A- 2009 090 383
- US-A1- 2010 153 076

## Description

### Technical Field

The present invention relates to a control apparatus of a manipulator system, a manipulator system, and a control method of a manipulator system.

### Background Art

In recent years, various proposals have been made for medical treatment technologies using robots. Especially in the surgical field, various proposals have been done concerning a surgical manipulator system that treats a patient using a multidegree-of-freedom manipulator having multidegree-of-freedom arms. For example, Japanese Patent No. 3680050 discloses the following technique.

That is, Japanese Patent No. 3680050 discloses a surgical manipulator comprising a working unit configured to perform a procedure, a manipulation unit configured to generate a manipulation instruction, a driving unit configured to drive the working unit based on the manipulation instruction of the manipulation unit, a power transmission mechanism configured to transmit the driving force of the driving unit to the working unit, and a control unit configured to control the driving unit based on the manipulation instruction of the manipulation unit. In this surgical manipulator, the power transmission mechanism includes a first power transmission unit provided on the side of the driving unit, a reduction gear provided between the first power transmission unit and the driving unit, and a second power transmission unit that is provided on the side of the working unit and is freely connectable/disconnectable to/from the first power transmission unit.

To reactivate the surgical manipulator system after its forced termination (termination caused not by a normal termination processing procedure, for example, termination that occurs when turning on an emergency stop switch or termination caused by sudden power shutdown), certain processing such as initialization of the surgical manipulator system is necessary, as a matter of course. In this case, the operator of the surgical manipulator system needs to know whether the termination in preceding use is forced termination or normal termination (termination other than forced termination, for example, termination by the normal termination processing procedure).

The surgical manipulator system disclosed in Japanese Patent No. 3680050 is configured to allow the user to determine whether the preceding termination is forced termination or normal termination. More specifically, this determination is done based on whether the surgical manipulator has been terminated with its joints being straightened.

Hence, if the preceding termination state of the surgical manipulator does not allow to clearly determine the states of the joints of the surgical manipulator (for example, if the joints are slightly bent), the determination cannot be done correctly. In addition, the surgical manipulator may have been terminated by forced termination even if its joints are straightened.

Under these circumstances, to apply the technique disclosed in Japanese Patent No. 3680050, the termination form (whether termination is forced termination or normal termination) of the surgical manipulator needs to separately be managed using a logbook or the like every time its use ends. Since this management takes time, the technique is not easy to use.

Document JP 2009 090383 A discloses a method of easily returning a robot to the origin without increasing a load on a system when the robot is returned from a stop position to the operating origin position. The returning direction of the robot is set, for each block, in a matrix-like area map including an interference area in which the operation of the robot is interfered and an operation area in which the robot is operated. A return path from the stop position is set according to the set returning direction. According to the returning path, the robot is moved and finally returned to the origin. When the returning direction of the robot is set, the robot can be returned to the origin irrespective of the present position of the robot.

### Disclosure of Invention

The present invention has been made in consideration of the above-described situation, and has as its object to provide a control apparatus of a manipulator system, a manipulator system including the control apparatus, and a control method of a manipulator system, which improve the usability by saving time for use of the manipulator system.

In order to achieve the above object, a control apparatus of a manipulator system according to the first aspect of the present invention, is characterized by comprising the features of claim 1.

In order to achieve the above object, a manipulator system according to the second aspect of the present invention, is characterized by comprising the features of claim 6.

In order to achieve the above object, a control method of a manipulator system according to the third aspect of the present invention, is characterized by comprising the features of claim 8.

### Brief Description of Drawings

FIG. 1 is a view showing an example of the arrangement of a surgical manipulator system according to the first embodiment of the present invention.
FIG. 2 is a flowchart of operation control by the slave control circuit of the surgical manipulator system according to the first embodiment of the present invention.
FIG. 3 is a flowchart of operation control by the slave control circuit of a surgical manipulator system according to the second embodiment of the present invention.

### Mode for Carrying Out the Invention

The embodiments of the present invention will now be described with reference to the accompanying drawings.

### [First Embodiment]

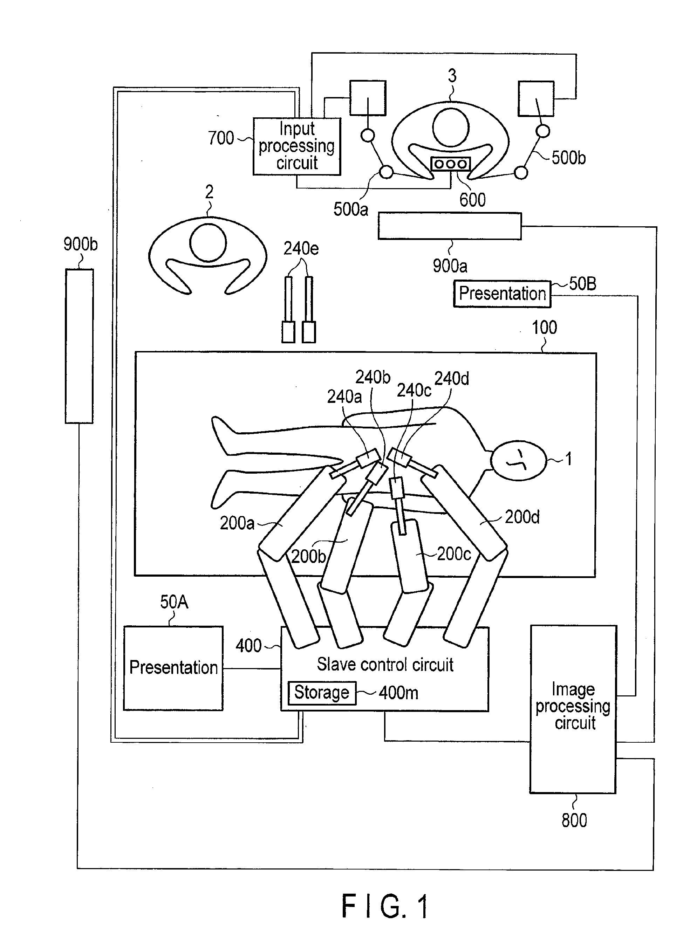

FIG. 1 is a view showing an example of the arrangement of a surgical manipulator system according to the first embodiment of the present invention. In the first embodiment, an example of application to a master-slave surgical manipulator system will be explained.

The master-slave surgical manipulator system is a system that includes two types of arms, that is, a master arm and a slave arm and remote-controls the slave arm such that it follows the operation of the master arm.

The surgical manipulator system shown in FIG. 1 includes an operating table 100, slave arms 200a, 200b, 200c, and 200d each serving as a working unit to perform a surgical procedure, a slave control circuit 400 serving as a control unit that controls the operations of the slave arms 200a, 200b, 200c, and 200d, master arms 500a and 500b, a manipulation unit 600 that generates a manipulation instruction, an input processing circuit 700, an image processing circuit 800, displays 900a and 900b, and presentation units 50A and 50B.

The operating table 100 is a table on which a patient 1 as an observation/treatment target is placed. The plurality of slave arms 200a, 200b, 200c, and 200d are installed near the operating table 100. Note that the slave arms 200a, 200b, 200c, and 200d may be installed on the operating table 100.

Each of the slave arms 200a, 200b, 200c, and 200d is a slave arm having a plurality of multidegree-of-freedom joints. The slave arms 200a, 200b, 200c, and 200d position various kinds of instruments such as a surgical instrument and an observation device attached to the sides of the distal ends of the slave arms 200a, 200b, 200c, and 200d (the sides closer to the body cavity of the patient 1) relative to the patient 1 placed on the operating table 100 by bending the joints.

The multidegree-of-freedom joints of the slave arms 200a, 200b, 200c, and 200d are individually driven by power units provided in the respective arms. As each power unit, for example, a motor (servo motor) including a servo mechanism with an incremental encoder or a reduction gear is used. Operation control of the servo motor is done by an operation control unit (not shown) of the slave control circuit 400.

The slave arms 200a, 200b, 200c, and 200d also include a plurality of power units configured to drive instruments 240a, 240b, 240c, and 240d attached to the sides of the distal ends of the slave arms. As each power unit as well, for example, a servo motor is used. Operation control of this servo motor is also done by the operation control unit (not shown) of the slave control circuit 400.

When the power units of the slave arms 200a, 200b, 200c, and 200d are driven, each motor driving amount is detected by a position detector. A detection signal output from each position detector is input to the slave control circuit 400. The slave control circuit 400 detects the driving amounts of the slave arms 200a, 200b, 200c, and 200d based on the detection signals.

Each of the instruments 240a, 240b, 240c, and 240d constitutes a working unit that performs a surgical procedure, includes joints having multiple degrees of freedom, and is inserted into the body cavity of the patient 1 from an insertion opening (not shown) formed in the body wall of the patient 1. The distal end of each of the instruments 240a, 240b, 240c, and 240d is configured to be bent and rotated.

The bending is performed by, for example, driving the servo motors provided in the slave arms 200a, 200b, 200c, and 200d to push-pull wires inserted in the instruments 240a, 240b, 240c, and 240d.

The rotation is performed by, for example, driving the servo motors provided in the slave arms 200a, 200b, 200c, and 200d to actuate rotation mechanisms provided in the instruments 240a, 240b, 240c, and 240d. Some kinds of instruments have opening/closing mechanisms at the distal ends. The opening/closing mechanisms are also actuated by, for example, driving the servo motors provided in the slave arms 200a, 200b, 200c, and 200d to push-pull the wires inserted in the instruments.

Note that in FIG. 1, reference numeral 240e denotes a replacement instrument which is configured to be interchangeable with the above-described instruments 240a to 240d. The instrument exchange operation is performed by, for example, an assistant 2.

In the first embodiment, assume that, for example, the slave arms 200a, 200b, and 200d out of the four slave arms 200a, 200b, 200c, and 200d shown in FIG. 1 are used as surgical slave arms. In this case, various kinds of surgical instruments are attached to the slave arms 200a, 200b, and 200d as the instruments 240a, 240b, and 240d.

Note that the surgical instrument in the first embodiment means an instrument such as a knife, scissors, or a retractor used to perform surgery or manipulation on a tissue part in the body of the patient 1. When the slave arm 200c is used as a camera arm for observation, various kinds of observation devices are attached to the slave arm 200c as the instrument 240c. In the first embodiment, the observation device means an instrument such as an electronic endoscope used to observe a tissue part in the body of the patient 1. The instrument exchange operation is performed by, for example, the assistant 2.

The slave control circuit 400 is a control device that constitutes as a control means including, for example, a CPU and a memory. The slave control circuit 400 stores a predetermined program for controlling the slave arms 200a, 200b, 200c, and 200d. The slave control circuit 400 controls the operations of the slave arms 200a, 200b, 200c, and 200d and/or the instruments 240a, 240b, 240c, and 240d in accordance with control signals from the input processing circuit 700.

More specifically, the slave control circuit 400 specifies the manipulation target slave arm (or instrument) of a master arm manipulated by an operator 3 based on a control signal from the input processing circuit 700, and calculates a driving amount necessary for causing the specified slave arm (or instrument) to make a motion corresponding to the manipulation amount of the master arm by the operator 3.

The slave control circuit 400 controls the operation of the manipulation target slave arm based on the calculated driving amount. At the time, the slave control circuit 400 inputs a driving signal to the manipulation target slave arm. The slave control circuit 400 simultaneously controls the magnitude and polarity of the driving signal based on a detection signal input from the position detector of the power unit in accordance with the operation of the manipulation target slave arm such that the driving amount of the manipulation target slave arm equals the target driving amount.

Upon receiving an image signal input from the observation device attached to the slave arm 200c, the slave control circuit 400 outputs the input image signal to the image processing circuit 800.

The slave control circuit 400 includes a storage unit 400m. The slave control circuit 400 includes a position/orientation information generation unit (not shown) which generates information (to be referred to as position/orientation information hereinafter) about the position/orientation of each of the slave arms and instruments at the time of termination of the surgical manipulator system. Pieces of position/orientation information are generated by the position/orientation information generation unit and written in the storage unit 400m. The slave control circuit 400 also includes an information generation unit (not shown) which generates information (to be referred to as termination identification information hereinafter) representing the termination form of the surgical manipulator system at the time of termination. Termination identification information is generated by the information generation unit and written in the storage unit 400m. More specifically, the termination identification information is, for example, a flag representing termination by forced termination or a flag representing termination by normal termination. The storage unit 400m is a storage device that can perform both read and write and also hold information even during rest of the surgical manipulator system. Examples of the storage unit 400m are a hard disk and a nonvolatile memory.

Note that the storage unit 400m need not always be provided in the slave control circuit 400 and may be provided, for example, separately from the slave control circuit 400, as a matter of course.

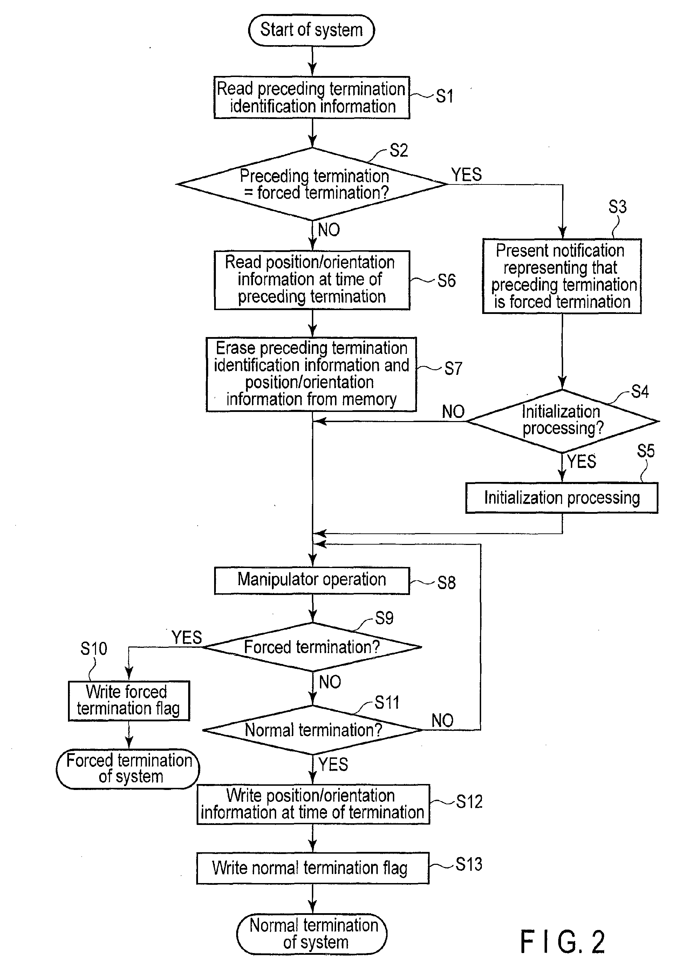

At the time of activation of the surgical manipulator system, the slave control circuit 400 reads out the position/orientation information and the termination identification information, determines whether the preceding termination is forced termination, and executes predetermined processing (processing according to the flowchart shown in FIG. 2 to be described later) based on the determination result. That is, the slave control circuit 400 includes a read unit (not shown) that reads out the termination identification information from the storage unit 400m at the time of activation of the surgical manipulator system, and a determination unit (not shown).

The presentation means 50A that constitutes the presentation unit is a sound output means/light-emitting means (for example, a speaker and a light) that outputs sound or emits light under the operation control of the slave control circuit 400 so as to cause the operator 3 or the assistant 2 to recognize that the preceding termination is forced termination.

The presentation means 50B that constitutes the presentation unit is formed from, for example, a liquid crystal display. If the preceding termination of the surgical manipulator system is forced termination, information that causes the operator 3 or the assistant 2 to recognize it is input from the slave control circuit 400 to the image processing circuit 800 at the next time of activation. The image processing circuit 800 generates image data corresponding to the information and outputs it to the presentation means 50B. The presentation means 50B displays the "image data that causes the operator 3 or the assistant 2 to recognize that the preceding termination is forced termination" input from the image processing circuit 800.

The master arms 500a and 500b include a plurality of link mechanisms. Each link of the link mechanisms is provided with a position detector such as an incremental encoder. The position detectors detect the operations of the links so that the input processing circuit 700 detects the manipulation amounts of the master arms 500a and 500b.

Note that in the example shown in FIG. 1, the master arm 500a is the arm manipulated by the right hand of the operator 3, and the master arm 500b is the arm manipulated by the left hand of the operator 3. That is, FIG. 1 illustrates an example in which the four slave arms are manipulated using the two master arms 500a and 500b. In this arrangement, the manipulation target slave arm of each master arm needs to be switched appropriately. The switching is done by, for example, manipulation of the manipulation unit 600 by the operator 3.

Note that the switching is unnecessary when equal numbers of master arms and slave arms are provided, and the master arms and their manipulation targets correspond in a one-to-one relationship.

The manipulation unit 600 includes various kinds of manipulation members such as switching buttons (to be simply referred to as switching buttons hereinafter) used to switch the manipulation target slave arms of the master arms 500a and 500b, scaling change switches that change the operation ratios between the master arms and the slave arms, and a foot switch used for emergency stop of the system. If the operator 3 has manipulated any one of the manipulation members of the manipulation unit 600, a manipulation signal according to the manipulation of the corresponding manipulation member is input from the manipulation unit 600 to the input processing circuit 700.

The input processing circuit 700 analyzes the manipulation signals from the master arms 500a and 500b and the manipulation signal (manipulation instruction) from the manipulation unit 600, generates a control signal to control the surgical manipulator system in accordance with the analysis result of the manipulation signals, and inputs the control signal to the slave control circuit 400.

The image processing circuit 800 performs various kinds of image processing for displaying the image signal input from the slave control circuit 400 to generate display image data for the operator display 900a and the assistant display 900b.

When the preceding termination of the surgical manipulator system is forced termination, an image signal that causes the operator 3 or the assistant 2 to recognize it is input from the slave control circuit 400 to the image processing circuit 800 at the next time of activation. The image processing circuit 800 performs predetermined image processing for the image signal to generate image data, and outputs it to the operator display 900a and the assistant display 900b.

Each of the operator display 900a and the assistant display 900b is formed from, for example, a liquid crystal display and displays an image based on image data generated by the image processing circuit 800 in accordance with an image signal acquired via an observation device. When the preceding termination is forced termination, the operator display 900a and the assistant display 900b receive, from the image processing circuit 800, image data that causes the operator 3 or the assistant 2 to recognize it, and display the image data.

FIG. 2 is a flowchart of operation control (processing concerning activation/termination of the surgical manipulator system) by the slave control circuit 400 of the surgical manipulator system according to the first embodiment. Note that the detailed operation of each unit of the surgical manipulator system is the same as described above, and the procedure of the processing will mainly be explained here.

When the surgical manipulator system is activated, indispensable initialization processing (not shown) such as system check is performed. The slave control circuit 400 then reads termination identification information from the storage unit 400m (step S1) and determines whether the preceding termination of the surgical manipulator system is forced termination (step S2). More specifically, if a flag representing forced termination is read from the storage unit 400m, the slave control circuit 400 determines that the preceding termination of the surgical manipulator system is forced termination (step S2 branches to YES). On the other hand, if a flag representing normal termination is read from the storage unit 400m, the slave control circuit 400 determines that the preceding termination of the surgical manipulator system is normal termination (step S2 branches to NO).

When step S2 braches to YES, the slave control circuit 400 performs operation control of the presentation means 50A and causes it to do predetermined sound output and/or light emission "to cause the user to recognize that the preceding termination of the surgical manipulator system is forced termination" (step S3). In step S3, the slave control circuit 400 also causes the image processing circuit 800 to generate image data "representing that the preceding termination of the surgical manipulator system is forced termination" and causes the presentation means 50B, the operator display 900a, and the assistant display 900b to display the image data.

Note that at the same time as (or instead of) presenting, to the operator 3 and the assistant 2, a notification representing that the preceding termination of the surgical manipulator system is forced termination in step S3, the slave control circuit 400 may perform processing of stopping the operation of the surgical manipulator system, processing of withdrawing the instruments to the extraction positions (the positions of instruments removed from body cavity of the patient 1), or the like, as a matter of course.

When the processing in step S3 has ended, the slave control circuit 400 determines whether a control signal to execute initialization processing (for example, movable range confirmation processing or origin detection processing that is normally dispensable initialization processing in addition to system check processing that is indispensable initialization processing) is transmitted from the input processing circuit 700 (step S4). In other words, step S4 is the step of determining whether the operator 3 has performed the operation of initializing the surgical manipulator system. That is, the slave control circuit 400 includes an initialization processing unit (not shown) that determines, after the presentation processing by the presentation means 50A and 50B, whether the operation of initializing the surgical manipulator system has been performed, and upon determining that the operation has been performed, initializes the surgical manipulator system.

Initialization processing is performed only after the operator 3 has performed the operation of performing initialization processing. This allows to prevent the slave arms 200a, 200b, 200c, and 200d and the instruments 240a, 240b, 240c, and 240d from operating independently of the intention of the operator 3 (against the intention of the operator 3). However, the initialization processing may forcibly be executed without performing the processing in step S4.

When step S4 braches to YES, the slave control circuit 400 executes the above-described initialization processing (step S5). After the end of the processing in step S5, or when step S4 braches to NO, the process advances to step S8 to be described later. Step S4 braches to NO in, for example, an urgent situation in which not a moment is to be lost. For example, when the operator or technician (not shown) determines that the manipulation of the manipulator is necessary and possible without initialization processing, an operation for branching step S4 to NO is performed to execute such a manipulator operation.

When step S2 branches to NO (when the preceding termination of the surgical manipulator system is normal termination), the slave control circuit 400 reads position/orientation information from the storage unit 400m (step S6). Next, the pieces of information already read from the storage unit 400m (the termination identification information read in step S1 and the position/orientation information read in step S6) are erased from the storage unit 400m (step S7).

Note that although not illustrated in the flowchart of FIG. 2, when the position/orientation information read in step S6 is appropriately used in, for example, initialization processing such as origin detection processing before the start of operation control in step S8 to be described later (or at the time of the start), the origin detection processing and the like need not be performed each time at the start of the system. This allows to shorten the activation time and improve the convenience. In addition, preferably, an appropriate moderating ratio is set in the driving unit to prevent the slave arms or instruments from operating in the power-off state, or the slave arms or instruments are designed and managed not to collide against other devices or persons at the time of movement or accommodation.

After the processing according to the preceding termination state of the surgical manipulator system has been performed in steps S1 to S7 described above, the slave control circuit 400 starts operation control of the slave arms 200a, 200b, 200c, and 200d and the instruments 240a, 240b, 240c, and 240d in accordance with the manipulation of the manipulation unit 600 by the operator 3 (step S8).

After the start of operation control in step S8, the slave control circuit 400 determines whether to do forced termination of the surgical manipulator system (step S9). When step S9 braches to YES, the slave control circuit 400 writes a flag representing forced termination in the storage unit 400m as termination identification information (step S10) and terminates the surgical manipulator system.

When step S9 braches to NO, the slave control circuit 400 determines whether to do normal termination of the surgical manipulator system (step S11). When step S11 braches to NO, the process returns to step S8.

On the other hand, when step S11 branches to YES, the slave control circuit 400 writes position/orientation information at this point of time in the storage unit 400m (step S12). Writing the position/orientation information in the storage unit 400m in step S12 makes it possible to omit the initialization processing such as origin detection processing at the next time of activation.

Next, the slave control circuit 400 writes a flag representing normal termination in the storage unit 400m as termination identification information (step S13) and terminates the surgical manipulator system.

As described above, according to the first embodiment, it is possible to provide a control apparatus of a manipulator system, a manipulator system including the control apparatus, and a control method of a manipulator system, which improve the usability by saving time for use of the manipulator system.

### [Second Embodiment]

A surgical manipulator system according to the second embodiment of the present invention will be described below. Differences from the first embodiment will be explained to avoid a repetitive description. One of the major differences between the first embodiment and the second embodiment is the presence/absence of a flag representing forced termination.

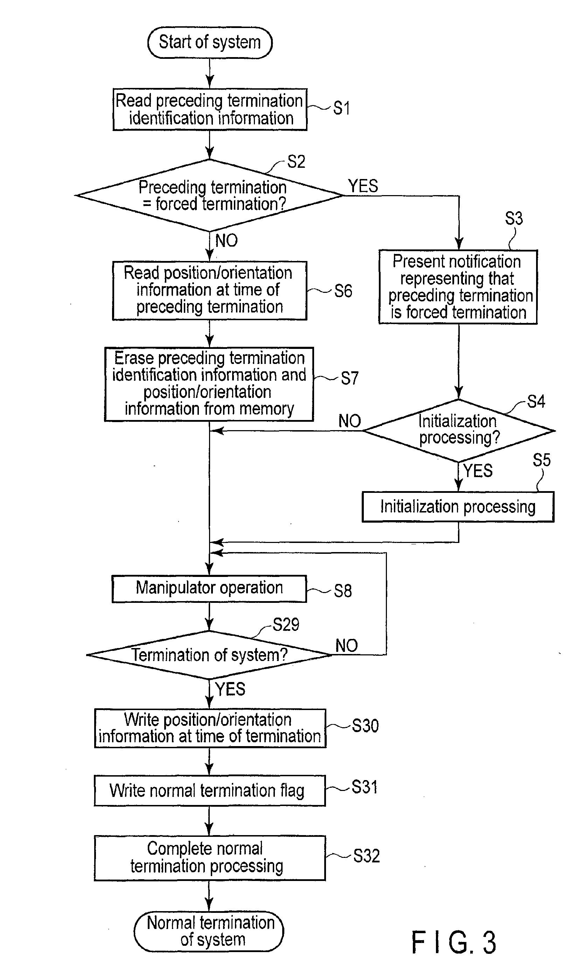

FIG. 3 is a flowchart of operation control by a slave control circuit 400 of a surgical manipulator system according to the second embodiment of the present invention. The same step numbers as in the flowchart of FIG. 2 denote steps in which the same processes is performed in the flowchart of FIG. 3.

After the start of operation control of the slave arms and instruments in step S8, the slave control circuit 400 determines whether to do normal termination of the surgical manipulator system (step S29). When step S29 braches to NO, the process returns to step S8.

On the other hand, when step S29 branches to YES, the slave control circuit 400 writes position/orientation information at this point of time in a storage unit 400m (step S30). Writing the position/orientation information in the storage unit 400m in step S30 makes it possible to omit the initialization processing such as origin detection processing at the next time of activation.

Next, the slave control circuit 400 generates a flag representing normal termination and writes it in the storage unit 400m as termination identification information (step S31) and terminates the surgical manipulator system (step S32).

In the surgical manipulator system according to the second embodiment, the above-described termination processing is performed (the flag representing forced termination is not generated). For this reason, when determining the preceding termination form in step S2, it is determined that the preceding termination is forced termination if the flag representing normal termination is not read in step S1.

As described above, according to the second embodiment, it is possible to provide a control apparatus of a manipulator system, a manipulator system including the control apparatus, and a control method of a manipulator system, which have the same effects as those of the control apparatus of a manipulator system, the manipulator system including the control apparatus, and the control method of a manipulator system according to the first embodiment.

The present invention has been described above based on the embodiments. However, the present invention is not limited to the above-described embodiments, and various modifications and applications can be made without departing from the spirit and scope of the invention.

The above-described embodiments incorporate inventions of various stages, and various inventions can be extracted by appropriately combining a plurality of constituent elements disclosed in the embodiments. For example, even when several constituent elements are omitted from all the constituent elements described in the embodiments, the arrangement without the omitted constituent elements can also be extracted as an invention if the above-described problems can be solved, and the above-described effects can be obtained.

## Claims

1. A control apparatus of a manipulator system, comprising:
a working unit (200a, 200b, 200c, 200d) configured to perform a procedure;
an information generation unit configured to generate termination identification information representing a termination form at the time of termination of the manipulator system;
a position/orientation information generation unit configured to generate position/orientation information of the working unit (200a, 200b, 200c, 200d) at the time of termination of the manipulator system;
a storage unit (400m) configured to store the termination identification information and the position/orientation information;
a read unit configured to read the termination identification information at the time of activation of the manipulator system; and
a control unit (400) configured to cause presentation of notification representing that the termination is a forced termination, perform processing of stopping operation of the manipulator system, or perform processing of withdrawing instruments of the manipulator system to extraction positions, when the termination identification information obtained by a preceding reading is determined to indicate forced termination, **characterized in that**
the read unit is further configured to read the position/orientation information when the termination identification information obtained by a preceding reading is determined to indicate normal termination, and
the storage unit (400m) is configured to erase the stored termination identification information and position/orientation information.

2. The control apparatus of the manipulator system according to claim 1,
**characterized by** further comprising:
a determination unit configured to determine, at the time of activation of the manipulator system based on the termination identification information read by the reading unit, whether a preceding termination form of the manipulator system is forced termination; and
a presentation unit (50A, 50B) configured to, when the determination unit has determined that the preceding termination form of the manipulator system is forced termination, present a notification of the forced termination to an operator of the manipulator system.

3. The control apparatus of the manipulator system according to claim 2, **characterized by** further comprising an initialization processing unit configured to determine, after presentation by the presentation unit (50A, 50B), whether an operation of initializing the surgical manipulator system has been performed, and upon determining that the operation has been performed, initialize the surgical manipulator system.

4. The control apparatus of the manipulator system according to claim 1, **characterized in that**
the manipulator system is a master-slave manipulator system, and
the storage unit (400m) is configured to store the termination identification information and the position/orientation information when an operation of terminating the surgical manipulator system has been performed.

5. The control apparatus of the manipulator system according to claim 1, **characterized in that** the termination identification information is one of a flag representing forced termination and a flag representing normal termination.

6. A manipulator system **characterized by** comprising:
a working unit (200a, 200b, 200c, 200d) configured to perform a procedure;
a manipulation unit (600) configured to generate a manipulation instruction; and
a control unit (400) configured to perform operation control of the working unit (200a, 200b, 200c, 200d) based on the manipulation instruction of the manipulation unit (600),
the control unit (400) comprising:
an information generation unit configured to generate termination identification information representing a termination form at the time of termination of the manipulator system;
a position/orientation information generation unit configured to generate position/orientation information of the working unit (200a, 200b, 200c, 200d) at the time of termination of the manipulator system;
a storage unit (400m) configured to store the termination identification information and the position/orientation information;
a read unit configured to read the termination identification information at the time of activation of the manipulator system; and
an operation control unit configured to control the working unit (200a, 200b, 200c, 200d) based on the termination identification information read by the reading unit and the manipulation instruction generated by the manipulation unit (600), wherein the control unit (400) is configured to cause presentation of notification representing that the termination is a forced termination, perform processing of stopping operation of the manipulator system, or perform processing of withdrawing instruments of the manipulator system to extraction positions, when the termination identification information obtained by a preceding reading is determined to indicate forced termination **characterized in that**
the operation control unit is configured to read the position/orientation information when it is determined that the termination identification information which has been read indicates normal termination, and control the working unit (200a, 200b, 200c, 200d),
the storage unit (400m) is configured to erase the stored termination identification information and position/orientation information, and

7. The manipulator system according to claim 6, **characterized in that** the termination identification information is one of a flag representing forced termination and a flag representing normal termination.

8. A control method of a manipulator system that has a working unit (200a, 200b, 200c, 200d) configured to perform a procedure, the control method being **characterized by** comprising:
generating, by an information generation unit, termination identification information representing a termination form at the time of termination of the manipulator system;
generating, by a position/orientation information generation unit, position/orientation information of the working unit (200a, 200b, 200c, 200d) at the time of termination of the manipulator system;
storing, by a storage unit (400m), the termination identification information and the position/orientation information;
reading, by a read unit, the termination identification information at the time of activation of the manipulator system;
causing, by a control unit (400), presentation of notification representing that the termination is a forced termination, processing of stopping operation of the manipulator system, or processing of withdrawing instruments of the manipulator system to extraction positions, when the termination identification information obtained by a preceding reading is determined to indicate forced termination; **characterized by**
reading, by the read unit, the position/orientation information when the termination identification information obtained by a preceding reading is determined to indicate normal termination; and
erasing, by the storage unit (400m), the stored termination identification information and position/orientation information.

9. The control method of the manipulator system according to claim 8, **characterized in that** the termination identification information is one of a flag representing forced termination and a flag representing normal termination.

## Patentansprüche

1. Steuervorrichtung eines Manipulatorsystems, umfassend:
eine Arbeitseinheit (200a, 200b, 200c, 200d), die dazu ausgelegt ist, einen Arbeitsablauf auszuführen;
eine Informationserzeugungseinheit, die dazu ausgelegt ist, eine Anhaltefeststellungsinformation zu erzeugen, die eine Anhalteform zum Zeitpunkt des Anhaltens des Manipulatorsystems darstellt;
eine Positions-/Ausrichtungsinformations-Erzeugungseinheit, die dazu ausgelegt ist, Positions-/Ausrichtungsinformation der Arbeitseinheit (200a, 200b, 200c, 200d) zum Zeitpunkt des Anhaltens des Manipulatorsystems zu erzeugen;
eine Speichereinheit (400m), die dazu ausgelegt ist, die Anhaltefeststellungsinformation und die Positions-/Ausrichtungsinformation zu speichern;
eine Leseeinheit, die dazu ausgelegt ist, die Anhaltefeststellungsinformation zum Zeitpunkt der Aktivierung des Manipulatorsystems zu lesen; und
eine Steuereinheit (400), die dazu ausgelegt ist, die Präsentation einer Mitteilung zu verursachen, die angibt, dass es sich bei dem Anhalten um ein erzwungenes Anhalten handelt, die Verarbeitung des Abbruchs der Operation des Manipulatorsystems auszuführen oder die Verarbeitung des Zurückziehens von Instrumenten des Manipulatorsystems auf Rückzugpositionen auszuführen, wenn die aus einem vorherigen Lesevorgang erhaltene Anhaltefeststellungsinformation bestimmungsgemäß ein erzwungenes Anhalten anzeigt, **dadurch gekennzeichnet, dass**
die Leseeinheit darüber hinaus dazu ausgelegt ist, die Positions-/Aus-richtungsinformation zu lesen, wenn die aus einem vorherigen Lesevorgang erhaltene Anhaltefeststellungsinformation bestimmungsgemäß ein normales Anhalten anzeigt, und
die Speichereinheit (400m) dazu ausgelegt ist, die gespeicherte Anhaltefeststellungsinformation und die gespeicherte Positions-/Ausrichtungsinformation zu löschen.

2. Steuervorrichtung des Manipulatorsystems nach Anspruch 1, **dadurch gekennzeichnet, dass** sie darüber hinaus aufweist:
eine Bestimmungseinheit, die dazu ausgelegt ist, zum Zeitpunkt der Aktivierung des Manipulatorsystems auf der Grundlage der von der Leseeinheit gelesenen Anhaltefeststellungsinformation zu bestimmen, ob es sich bei einer vorherigen Anhalteform des Manipulatorsystems um ein erzwungenes Anhalten handelt; und
eine Präsentationseinheit (50A, 50B), die, wenn die Bestimmungseinheit bestimmt hat, dass es sich bei der vorherigen Anhalteform des Manipulatorsystems um ein erzwungenes Anhalten handelt, dazu ausgelegt ist, einem Bediener des Manipulatorsystems eine Mitteilung über das erzwungene Anhalten zu präsentieren.

3. Steuervorrichtung des Manipulatorsystems nach Anspruch 2, **dadurch gekennzeichnet, dass** sie des Weiteren eine Initialisierungsverarbeitungseinheit aufweist, die dazu ausgelegt ist, nach der Präsentation durch die Präsentationseinheit (50A, 50B) zu bestimmen, ob ein Vorgang der Initialisierung des chirurgischen Manipulatorsystems ausgeführt wurde, und nach der Bestimmung, dass der Vorgang ausgeführt wurde, das chirurgische Manipulatorsystem zu initialisieren.

4. Steuervorrichtung des Manipulatorsystems nach Anspruch 1, **dadurch gekennzeichnet, dass**
das Manipulatorsystem ein Master/Slave-Manipulatorsystem ist, und
die Speichereinheit (400m) dazu ausgelegt ist, die Anhaltefeststellungsinformation und die Positions-/Ausrichtungsinformation zu speichern, wenn ein Vorgang des Anhaltens des chirurgischen Manipulatorsystems ausgeführt wurde.

5. Steuervorrichtung des Manipulatorsystems nach Anspruch 1, **dadurch gekennzeichnet, dass** es sich bei der Anhaltefeststellungsinformation um einen Merker, der ein erzwungenes Anhalten anzeigt, oder einen Merker handelt, der ein normales Anhalten anzeigt.

6. Manipulatorsystem, **dadurch gekennzeichnet, dass** es umfasst:
eine Arbeitseinheit (200a, 200b, 200c, 200d), die dazu ausgelegt ist, einen Arbeitsablauf auszuführen;
eine Manipulationseinheit (600), die dazu ausgelegt ist, einen Manipulationsbefehl zu erzeugen; und
eine Steuereinheit (400), die dazu ausgelegt ist, eine Operationssteuerung der Arbeitseinheit (200a, 200b, 200c, 200d) auf der Grundlage des Manipulationsbefehls der Manipulationseinheit (600) auszuführen,
wobei die Steuereinheit (400) umfasst:
eine Informationserzeugungseinheit, die dazu ausgelegt ist, eine Anhaltefeststellungsinformation zu erzeugen, die eine Anhalteform zum Zeitpunkt des Anhaltens des Manipulatorsystems angibt;
eine Positions-/Ausrichtungsinformations-Erzeugungseinheit, die dazu ausgelegt ist, Positions-/Ausrichtungsinformation der Arbeitseinheit (200a, 200b, 200c, 200d) zum Zeitpunkt des Anhaltens des Manipulatorsystems zu erzeugen;
eine Speichereinheit (400m), die dazu ausgelegt ist, die Anhaltefeststellungsinformation und die Positions-/Ausrichtungsinformation zu speichern;
eine Leseeinheit, die dazu ausgelegt ist, die Anhaltefeststellungsinformation zum Zeitpunkt der Aktivierung des Manipulatorsystems zu lesen; und
eine Operationssteuereinheit, die dazu ausgelegt ist, die Arbeitseinheit (200a, 200b, 200c, 200d) auf der Grundlage der von der Leseeinheit gelesenen Anhaltefeststellungsinformation und dem von der Manipulationseinheit (600) erzeugten Manipulationsbefehl zu steuern, wobei die Steuereinheit (400) dazu ausgelegt ist, die Präsentation einer Mitteilung zu veranlassen, die angibt, dass es sich bei dem Anhalten um ein erzwungenes Anhalten handelt, die Verarbeitung des Abbruchs der Operation des Manipulatorsystems auszuführen oder die Verarbeitung des Zurückziehens von Instrumenten des Manipulatorsystems auf Rückzugpositionen auszuführen, wenn die durch einen vorherigen Lesevorgang erhaltene Anhaltefeststellungsinformation bestimmungsgemäß ein erzwungenes Anhalten anzeigt, **dadurch gekennzeichnet, dass**
die Operationssteuereinheit dazu ausgelegt ist, die Positions-/Ausrichtungsinformation zu lesen, wenn festgestellt wird, dass die gelesene Anhaltefeststellungsinformation ein normales Anhalten anzeigt, und die Arbeitseinheit (200a, 200b, 200c, 200d) zu steuern,
wobei die Steuereinheit (400m) dazu ausgelegt ist, die gespeicherte Anhaltefeststellungsinformation und die gespeicherten Positions/Ausrichtungsinformation zu löschen.

7. Manipulatorsystem nach Anspruch 6, **dadurch gekennzeichnet, dass** es sich bei der Anhaltefeststellungsinformation um einen Merker, der ein erzwungenes Anhalten anzeigt, oder einen Merker handelt, der ein normales Anhalten anzeigt.

8. Steuerverfahren eines Manipulatorsystems, das über eine Arbeitseinheit (200a, 200b, 200c, 200d) verfügt, die dazu ausgelegt ist, einen Arbeitsablauf auszuführen, wobei das Steuerverfahren **dadurch gekennzeichnet ist, dass** es umfasst:
Erzeugen, durch eine Informationserzeugungseinheit, einer Anhaltefeststellungsinformation, die eine Anhalteform zum Zeitpunkt des Anhaltens des Manipulatorsystems angibt;
Erzeugen, durch eine Positions-/Ausrichtungsinformations-Erzeugungseinheit, einer Positions-/Ausrichtungsinformation der Arbeitseinheit (200a, 200b, 200c, 200d) zum Zeitpunkt des Anhaltens des Manipulatorsystems;
Speichern, durch eine Speichereinheit (400m), der Anhaltefeststellungsinformation und der Positions-/Ausrichtungsinformation;
Lesen, durch eine Leseeinheit, der Anhaltefeststellungsinformation zum Zeitpunkt der Aktivierung des Manipulatorsystems;
Veranlassen, durch eine Steuereinheit (400), der Präsentation einer Mitteilung, die angibt, dass es sich bei dem Anhalten um ein erzwungenes Anhalten handelt, der Verarbeitung des Abbruchs der Operation des Manipulatorsystems oder der Verarbeitung des Zurückziehens von Instrumenten des Manipulatorsystems auf Rückzugpositionen, wenn die durch einen vorherigen Lesevorgang erhaltene Anhaltefeststellungsinformation bestimmungsgemäß ein erzwungenes Anhalten anzeigt; **gekennzeichnet durch**
Lesen, **durch** die Leseeinheit, der Positions-/Ausrichtungsinformation, wenn die **durch** einen vorherigen Lesevorgang erhaltene Anhaltefeststellungsinformation bestimmungsgemäß ein normales Anhalten anzeigt; und
Löschen, **durch** die Speichereinheit (400m), der gespeicherten Anhaltefeststellungsinformation und der gespeicherten Positions/Ausrichtungsinformationen.

9. Steuervorrichtung des Manipulatorsystems nach Anspruch 8, **dadurch gekennzeichnet, dass** es sich bei der Anhaltefeststellungsinformation um einen Merker, der ein erzwungenes Anhalten anzeigt, oder einen Merker handelt, der ein normales Anhalten anzeigt.

## Revendications

1. Appareil de commande d'un télémanipulateur, comprenant :
une unité de travail (200a, 200b, 200c, 200d) conçue pour exécuter une procédure ;
une unité génératrice d'informations conçue pour générer des informations d'identification d'arrêt représentant une forme d'arrêt à l'instant d'arrêt du télémanipulateur ;
une unité génératrice d'informations de position/d'orientation conçue pour générer des informations de position/d'orientation de l'unité de travail (200a, 200b, 200c, 200d) à l'instant d'arrêt du télémanipulateur ;
une unité de mémorisation (400m) conçue pour mémoriser les informations d'identification d'arrêt et les informations de position/d'orientation ;
une unité de lecture conçue pour lire les informations d'identification d'arrêt à l'instant d'activation du télémanipulateur ; et
une unité de commande (400) conçue pour provoquer une présentation de notification indiquant le fait que l'arrêt est un arrêt forcé, exécuter un traitement d'opération d'interruption du télémanipulateur, ou exécuter un traitement de retrait d'instruments du télémanipulateur à des positions d'extraction, lorsque les informations d'identification d'arrêt obtenues par une lecture précédente sont déterminées comme indiquant un arrêt forcé, **caractérisé en ce que**
l'unité de lecture est en outre conçue pour lire les informations de position/d'orientation lorsque les informations d'identification d'arrêt obtenues par une lecture précédente sont déterminées comme indiquant un arrêt normal, et
l'unité de mémorisation (400m) est conçue pour effacer les informations d'identification d'arrêt et les informations de position/d'orientation mémorisées.

2. Appareil de commande d'un télémanipulateur selon la revendication 1, **caractérisé en ce qu'**il comprend en outre :
une unité de détermination conçue pour déterminer, à l'instant d'activation du télémanipulateur, sur la base des informations d'identification d'arrêt lues par l'unité de lecture, si une forme d'arrêt précédente du télémanipulateur est un arrêt forcé ; et
une unité de présentation (50A, 50B) conçue pour, lorsque l'unité de détermination a déterminé que la forme d'arrêt précédente du télémanipulateur est un arrêt forcé, présenter une notification de l'arrêt forcé à un opérateur du télémanipulateur.

3. Appareil de commande d'un télémanipulateur selon la revendication 2, **caractérisé en ce qu'**il comprend en outre une unité de traitement d'initialisation conçue pour déterminer, après la présentation par l'unité de présentation (50A, 50B), si une opération d'initialisation du télémanipulateur chirurgical a été exécutée et, lors de la détermination du fait que l'opération a été exécutée, initialiser le télémanipulateur chirurgical.

4. Appareil de commande d'un télémanipulateur selon la revendication 1, **caractérisé en ce que**
le télémanipulateur est un télémanipulateur maître-esclave, et
l'unité de mémorisation (400m) est conçue pour mémoriser les informations d'identification d'arrêt et les informations de position/d'orientation lorsqu'une opération d'arrêt du télémanipulateur chirurgical a été exécutée.

5. Appareil de commande d'un télémanipulateur selon la revendication 1, **caractérisé en ce que** les informations d'identification d'arrêt constituent soit un drapeau représentant un arrêt forcé soit un drapeau représentant un arrêt normal.

6. Télémanipulateur **caractérisé en ce qu'**il comprend :
une unité de travail (200a, 200b, 200c, 200d) conçue pour exécuter une procédure ;
une unité de manipulation (600) conçue pour générer une instruction de manipulation ; et
une unité de commande (400) conçue pour exécuter une commande de mise en oeuvre de l'unité de travail (200a, 200b, 200c, 200d) sur la base de l'instruction de manipulation de l'unité de manipulation (600),
l'unité de commande (400) comprenant :
une unité génératrice d'informations conçue pour générer des informations d'identification d'arrêt représentant une forme d'arrêt à l'instant d'arrêt du télémanipulateur ;
une unité génératrice d'informations de position/d'orientation conçue pour générer des informations de position/d'orientation de l'unité de travail (200a, 200b, 200c, 200d) à l'instant d'arrêt du télémanipulateur ;
une unité de mémorisation (400m) conçue pour mémoriser les informations d'identification d'arrêt et les informations de position/d'orientation ;
une unité de lecture conçue pour lire les informations d'identification d'arrêt à l'instant d'activation du télémanipulateur ; et
une unité de commande de mise en oeuvre conçue pour commander l'unité de travail (200a, 200b, 200c, 200d) sur la base des informations d'identification d'arrêt lues par l'unité de lecture et de l'instruction de manipulation générée par l'unité de manipulation (600), dans lequel l'unité de commande (400) est conçue pour provoquer une présentation de notification indiquant que l'arrêt est un arrêt forcé, exécuter un traitement d'opération d'interruption du télémanipulateur, ou exécuter un traitement de retrait d'instruments du télémanipulateur à des positions d'extraction, lorsque les informations d'identification d'arrêt obtenues par une lecture précédente sont déterminées comme indiquant un arrêt forcé, **caractérisé en ce que**
l'unité de commande de mise en oeuvre est conçue pour lire les informations de position/d'orientation lorsqu'il est déterminé que les informations d'identification d'arrêt qui ont été lues indiquent un arrêt normal, et commander l'unité de travail (200a, 200b, 200c, 200d), et **en ce que**
l'unité de mémorisation (400m) est conçue pour effacer les informations d'identification d'arrêt et les informations de position/d'orientation mémorisées.

7. Télémanipulateur selon la revendication 6, **caractérisé en ce que** les informations d'identification d'arrêt constituent soit un drapeau représentant un arrêt forcé soit un drapeau représentant un arrêt normal.

8. Procédé de commande d'un télémanipulateur qui comporte une unité de travail (200a, 200b, 200c, 200d) conçue pour exécuter une procédure, le procédé de commande étant **caractérisé en ce qu'**il consiste à :
générer, au moyen d'une unité génératrice d'informations, des informations d'identification d'arrêt représentant une forme d'arrêt à l'instant d'arrêt du télémanipulateur ;
générer, au moyen d'une unité génératrice d'informations de position/d'orientation, des informations de position/d'orientation de l'unité de travail (200a, 200b, 200c, 200d) à l'instant d'arrêt du télémanipulateur ;
mémoriser, au moyen d'une unité de mémorisation (400m), les informations d'identification d'arrêt et les informations de position/d'orientation ;
lire, par une unité de lecture, les informations d'identification d'arrêt à l'instant d'activation du télémanipulateur ;
provoquer, au moyen d'une unité de commande (400), une présentation de notification indiquant que l'arrêt est un arrêt forcé, un traitement d'opération d'interruption du télémanipulateur, ou un traitement de retrait d'instruments du télémanipulateur à des positions d'extraction, lorsque les informations d'identification d'arrêt obtenues par une lecture précédente sont déterminées comme indiquant un arrêt forcé ; **caractérisé par**
la lecture, au moyen de l'unité de lecture, des informations de position/d'orientation lorsque les informations d'identification d'arrêt obtenues par une lecture précédente sont déterminées comme indiquant un arrêt normal ; et
l'effacement, au moyen de l'unité de mémorisation (400m), des informations d'identification d'arrêt et des informations de position/d'orientation mémorisées.

9. Procédé de commande de télémanipulateur selon la revendication 8, **caractérisé en ce que** les informations d'identification d'arrêt constituent soit un drapeau représentant un arrêt forcé soit un drapeau représentant un arrêt normal.
